# EUROPEAN PATENT APPLICATION

(11) **EP 1 062 925 A1**
(43) Date of publication of application: **27.12.2000**
(21) Application number: 99305440.2
(22) Date of filing: 08.07.1999
(51) Int. Cl.: A61F 5/02

(54) **A pelvis support**

(30) Priority: 08.07.1998 GB 9814837
(71) Applicant: Langley E & D Limited, London NW2 4SE (GB)
(72) Inventor: Langley, Nicholas, London NW2 4SE (GB)
(74) Representative: Roberts, Gwilym Vaughan

(57) **Abstract**

A pelvis support comprises a retainer 17 carrying a plurality of wedges 10,11 and arranged to be worn by a wearer. The wedges are shaped and positioned to cooperate with an external surface against which the wearer is resting to support the pelvis.

## Description

This invention relates to a pelvis support, in particular a device to prevent detrimental natural rotation of the pelvis and the resulting damage to the ligaments and intervertebral disc between the transitional vertebrae, when the human body is in a relaxed state. By preventing this natural rotation of the pelvis, the invention will help prevent or alleviate many different types of back injury.

Back-ache relief devices that are currently available range from corsets to special mattresses. There are various problems associated with the known devices, in particular, devices such as body belts are regarded as medical appliances and are generally recommended to people who have serious back problems. Accordingly there is associated with such devices in the minds of the general public and in particular sufferers of back problems that known devices are only suitable or beneficial for those having serious back problems. Accordingly, many people suffering from back conditions will avoid the use of the known devices as they may be uncomfortable, complex, restricting, unwieldy and may not be successful or even may be therapeutically detrimental.

With regard to the body belts currently available, the devices are generally bulky or restrict natural body movement and hence are uncomfortable to wear. In addition many back support devices put pressure on the lumbar region of the spine purporting to improve the natural curvature of the back. In fact this can do more harm than good. Furthermore, the corset-like appearance and operation of known lumbar support belts is not suitable to many users for reasons of appearance, comfort, effectiveness and/or psychological factors. The corset relies on being tight-fitting around the abdomen which can result in damage to the stomach and various other organs in that region.

As a further option, it has been suggested to provide appropriately shaped or sprung mattresses. The mattresses are, however, costly and difficult to make, and unlikely to be suitable as they are generally not able to concentrate pressure on the required areas of the body especially as the user may change his or her sleeping position during the night.

GB 2259848 relates to a protective belt for use, for example, in motocross which is releasably fastened to the inside of suitable trousers. However this implementation is for protective rather than curative purposes and only for use in highly specific applications and especially is not designed to provide support while the body is in a relaxed state.

US 5,351,340 relates to a lumbar support member incorporated into a work garment such as a fire-fighter jacket. The support is intended to assist the user in carrying out strenuous activities in particular. Again the principle is not to provide support while the body is in a relaxed state.

The invention is set out in the appended claims.

Accordingly there is provided a therapeutic support for a wearer who is asleep or otherwise relaxing in a lying or sitting position. The support is unique in that it protects the spine from damage by supporting the pelvis. The support protects the spine by preventing the pelvis from natural rotation and consequential misalignment of the spine. The pelvis support itself relies for its support on an external surface such as a bed mattress or a chair back. The natural rotation of the pelvis and subsequent misalignment of the spine when the wearer is in a relaxed state are avoided. When the body is not in a relaxed state the muscles naturally tend to hold the pelvis in the correct position and the vertebrae properly aligned and so protect the area around the transitional vertebrae. Good body posture is important when standing especially as the invention is not designed to protect the spine without an external support.

The most basic means for mounting the support elements to prevent their movement with respect to the body may comprise a belt. The support elements are placed at the side and back body positions of the belt for use in the lying position whether the wearer is lying on his or her side or back. The support elements may only be placed at the back body positions of the belt for day-time use, in particular when the user is seated. The support elements are interrupted at the centre of back of the belt to provide clearance for the user's spine and avoid any pressure on that area of the body.

The support elements may be removable from the mounting means to allow ease of cleaning and versatility. The support elements may be inflatable allowing a desired dimension/resilience to be achieved. The support elements may be mounted in a garment for the wearer so as to be retained firmly but inconspicuously in place.

The support elements comprise generally wedge shaped elements provided as an integral part of the belt or garment and located to extend laterally around the top section of the wearer's pelvis. Optimum pelvis support is thus achieved, regardless of the wearer's position if lying down. A layer of firm material may extend around at least the inside of the wedge shaped elements for retaining them in place with respect to the body. A layer of soft material and a layer of wadding may be provided at the innermost part of the belt or garment for increased comfort to the user. In particular, the soft material can absorb or prevent perspiration at the wearer's back in contact with the firm material, which may be non-porous. The soft material may replace the firm material if it is able to maintain the belt or garment in the correct position.

The pelvis support element may be sewn into the belt or garment or the element may be retained in a pocket provided in the garment. In the latter case, the element can be replaced in the pocket with an element of different shape for different support requirements. The pocket may be configured to allow the insertion of different sized elements to achieve the desired thickness.

The pelvis support element may comprise inflatable elements, or elements that have been pre-inflated, allowing considerable weight reductions, and the inflatable element may comprise two, four or more inflatable compartments arranged to be located at the back and each side of the wearer. The individual compartments can be inflated to desired pressures to obtain optimum support.

Some or all of the elements of the garment may be constructed of disposable materials.

Embodiments of the invention will now be described by way of example with reference to the accompanying drawings in which:
Fig. 1A is a front view the human skeletal system which is vulnerable to damage as a result of natural rotation of the pelvis;
Fig. 1B is a side view corresponding to Fig. 1A without the hip and thigh bones shown as they hide the critical part from view in the side elevation;
Fig. 2A is a rear view of the bones in Fig. 1A shown in relation to the whole human body;
Fig. 2B is a side view corresponding to the rear view in fig. 2A;
Fig. 2C is the same view as Fig. 2A with the addition of the pelvis support wedges to illustrate their exact location in relation to the body. The rear wedges are shown in full while the side wedges are shown in section for clarity;
Fig. 2D is the same view as Fig. 2B also with the addition of the pelvis support wedges. In this case the side wedges are shown in full while the rear wedges are shown in section for clarity;
Fig. 3A shows the natural rotation of the pelvis and misalignments of the transitional vertebrae in the unsupported lying (side) position;
Fig. 3B shows the correct positioning of the pelvis and alignment of the transitional vertebrae in the lying (side) position when supported according to the present invention;
Fig. 4A shows the natural rotation of the pelvis and misalignment of the transitional vertebrae in the unsupported lying (back) position;
Fig. 4B shows the correct positioning of the pelvis and alignment of the transitional vertebrae in the lying (back) position when supported according to the present invention;
Fig. 5A shows the natural rotation of the pelvis and misalignment of the transitional vertebrae when in the unsupported sitting position;
Fig. 5B shows the correct positioning of the pelvis and alignment of the transitional vertebrae in the sitting position when supported according to the present invention;
Fig. 6A is a set of pelvis support wedges according to the present invention showing their positions in relation to each other for inclusion into a belt or garment;
Fig. 6B is an external view of a complete belt according to the present invention;
Fig. 6C shows the basic shapes of a side wedge.
Fig. 6D shows the basic shape of a pair of rear wedges for night time use.
Fig. 6E shows the basic shape of a pair of rear wedges for day time use.
Fig. 6F is an exploded top view of the components of a basic night-time pelvis support belt according to the present invention;
Fig. 6G is a non-exploded top view of the components in Fig. 6F including the positions of the stitching to attach the pockets to the belt;
Fig. 6H is the same as Fig. 6F with the addition of a layer of wadding and an extra fabric layer to keep it in place all to provide extra comfort;
Fig. 6J shows the components in Fig. 6H in non-exploded view;
Fig. 7A is an illustration of the pelvis support belt in Fig. 6B but for daytime use with only rear wedges sewn into pockets;
Fig. 7B is the same as Fig. 6B for night-time use with each rear and side wedge as a single integrated piece;
Fig. 7C is the same as Fig. 6B with each pocket sewn only on three sides and the fourth side held together with a Velcro™ type fastening;
Fig. 7D is the same as Fig. 6B with each pocket sewn only on three sides with fabric flaps over the fourth side of each pocket to hold the wedges in place;
Fig. 8 contains all the components and shows the main manufacturing steps to make the pelvis support belt similar to the one shown in Fig. 7D according to the present invention;
Fig. 9A shows night-time support wedges sewn into a pair of (boxer) shorts which has elastic inserts and Velcro™ type fastening at the front;
Fig. 9B is the same as Fig. 9A but for day-time use with only the rear wedges;
Fig. 9C is the same as Fig. 9A but with button type fastening at the front instead of Velcro™;
Fig. 9D is the same as Fig. 9A with the Velcro™ replaced by a continuous waist band containing the elastic inserts;

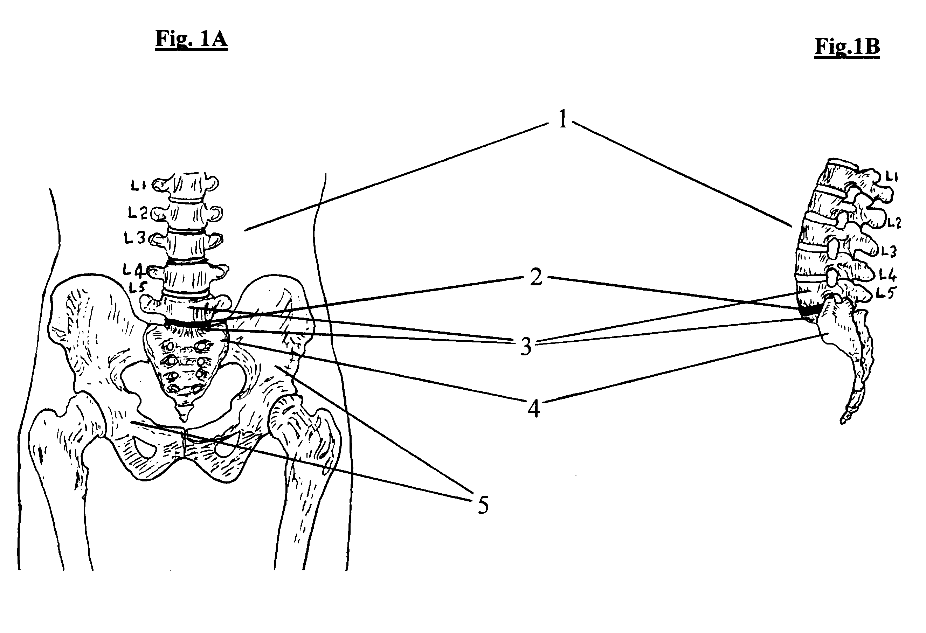

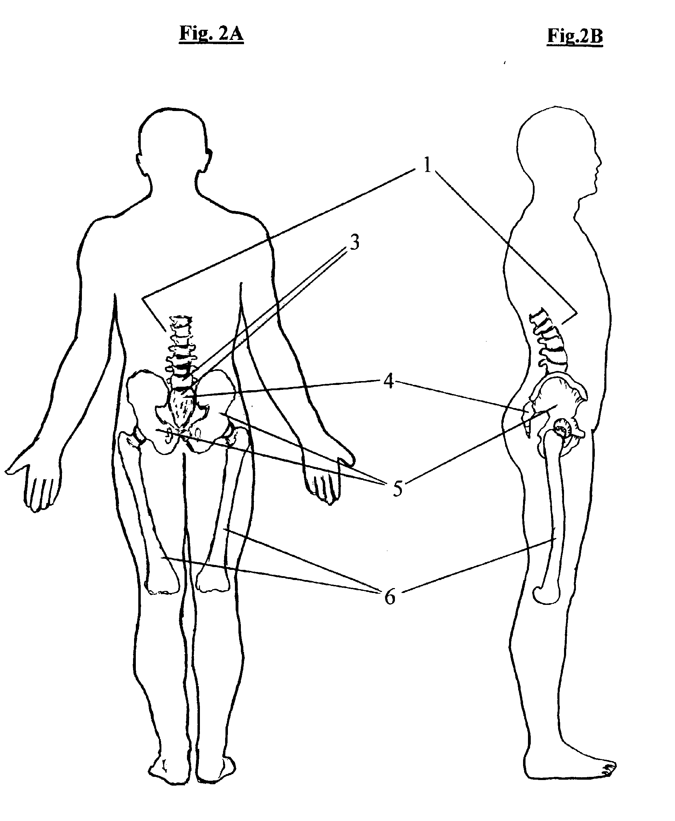

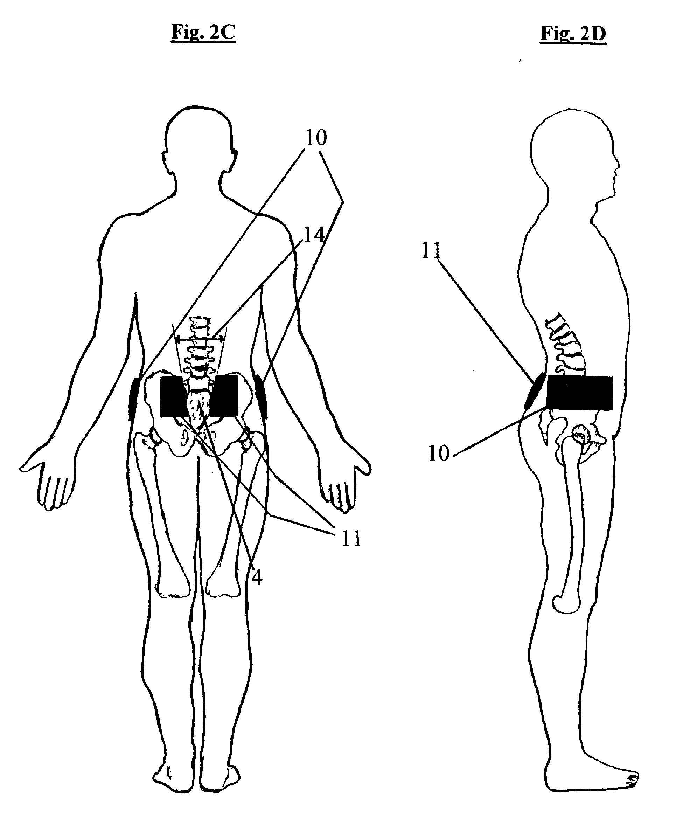

Referring to Figs. 1A/B and 2A/B the human skeletal system in the region of the pelvis 4 and 5 and spine 1 is constructed to provide the torso with maximum flexibility which makes it an exceptionally complex part of the body. The many bones, muscles, ligaments, cartilage and nerves are all susceptible to damage. This invention protects the part of the back most vulnerable to damage 2, when the body is in a relaxed state, which is in the region of the transitional vertebrae 3. These are the bottom vertebra L5 of the lumbar region 1 and the top vertebra of the sacrum 4. The vertebrae which form the sacrum 4 are fused together and form the keystone of a weight-bearing arch involving the hip bones 5. The two hip bones 5 and the sacrum 4 constitute the pelvis and are essentially a single structure. By contrast the lumbar region 1, as with most of the spine, is made up of vertebrae which move with respect to each other and provide the necessary flexibility of the torso. It is a widely proven fact that where a flexible cord is attached to a fixed object, the flexible section is most likely to fail immediately adjacent to the fixed object which, in the case of the spine, is the joint between the transitional vertebrae 3.

When the body is relaxed in a sleeping or sitting position, without the support and protection of the muscles, the pelvis 5 has a tendency to rotate naturally due to various forces and it is the transitional vertebrae 3 which absorb most of the resulting misalignment of the spine. Without the protection of the muscles, it is the ligaments and intervertebral disc 2 between the transitional vertebrae 3, which, as the last line of defence, prevent them going past the point beyond which they would become dislocated. It is this condition, where the ligaments are stretched to the limit and the intervertebral disc 2 and nerves are pinched, that damage and inflammation occur especially when this condition remains for long periods, such as during sleep or on long car journeys. As discussed below in more detail this condition is avoided according to the present invention by preventing rotation of the pelvis and the subsequent misalignment of the transitional vertebrae when the body is in a relaxed mode.

The continuous stretching of the ligaments to their limit and the pinching of the intervertebral disc 2 and nerves, for extended periods frequently results in damage and inflammation in the vicinity. This damage can become permanent if it is never allowed enough time to recover. It results in discomfort which is then exaggerated by sitting in one position for long periods, such as during car journeys. In other cases it can be exacerbated by actions such as a poor lifting technique which can cause cramp in the tiny local muscles and can be totally debilitating. Yet another consequence of the low level inflammation is the natural compensation, by other muscles in the back, in order to alleviate the discomfort. This often produces other back complaints, sometimes as far away as the neck, and can ultimately result in permanent posture deformities.

The natural instinct in the case of a serious back problem, is to lie on a bed, sometimes with pillows mistakenly supporting the lumbar region. It is unlikely that the pelvis 5 will be supported properly such that it allows the transitional vertebrae 3 region to recover properly. Thus, even with the help of anti-inflammatory drugs, recovery can take one or two weeks when it should only take a day or so. Furthermore, even after that period the back is not repaired and, unhappily, may never be completely repaired without the protection of the invention.

Referring to Figs. 2A to 2D, the present invention has identified the above problems and solves them by introducing pelvis support wedges 10 and 11. The top of the wedges are lined up with the top of the pelvis 5. The rear wedges 11 are positioned with a gap 14 between them which ensures that the support pressure is exerted on the pelvis hip bones 5 and not the sacrum 4. The side wedges 10 function when the body is lying on its side while the rear wedges 11 function when the body is lying on its back or sitting in a chair.

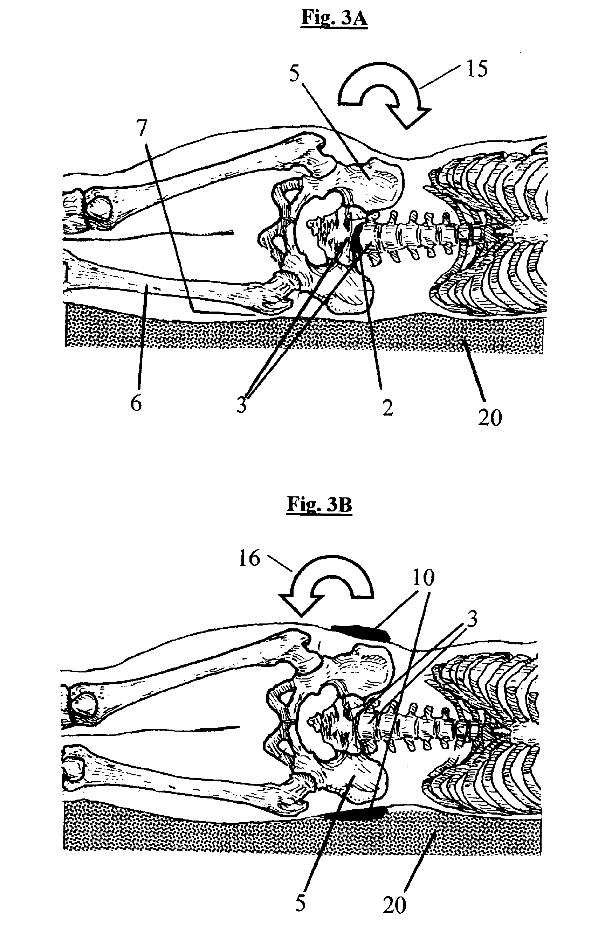

In its simplest form, the invention comprises the pelvis support wedges 10 and 11 incorporated in a belt. Together they co-operate with the body and an external supporting surface such as a mattress 20 or the back of a chair 21 to counteract misalignments of the spine which occur when the body is resting as illustrate by Figs. 3A/B, 4A/B and 5A/B. Although it is possible to use a single support belt whether the body is in a sitting or lying position, preferably different support belts are provided for day-time (sitting) and night-time (lying) use, each meeting the specific requirements of the respective mode of use. A common feature of the two applications is the pair of rear wedges 11 which have a similar function whether a body position lying on the back or sitting is adopted.

Another common feature of the pelvis support belt for both the sleeping and sitting positions is that they rely on a co-operating support for their correct operation. Referring to Figs. 3A and 3B, in the sleeping position the belt is designed to take pressure from the mattress 20 in order to provide the support.

Referring to Fig. 3A the natural pelvis 5 rotation 15 is caused when the body is in the sleeping (side) position by the eccentric pressure exerted by the femur (thigh) bone 6 through the hip joint 7. The misalignment of the pelvis 5 mainly affects the first flexible joint of the spine which is between the transitional vertebrae 3. Thus when the body is relaxed and the muscles are not providing any support, on one side the cartilage 2 is pinched and on the other side the ligaments are stretched to the limit.

Referring to Fig. 3B, the pelvis 5 is in a properly aligned position 16 as a result of wearing the invention 10 when the body is in the sleeping (side) position. The side wedge 10 is designed to take pressure from the mattress 20 in order to provide the support and hence allow the transitional vertebrae 3 to be properly aligned.

In the case of the night-time side wedges 10, a further therapeutic advantage is provided. Normally when a body is asleep on its side, a large portion of the body weight is concentrated through the hip joint 7 because it protrudes. As more than a quarter of one's life is spent asleep, this represents a total of 10 or 20 years concentrated pressure on the hip joint for people who sleep on their sides. The invention spreads this weight across the pelvis 5 and absorbs most of the pressure. This will inevitably help some of the people who suffer from joint diseases such as arthritis and may even help people with other bone problems such as brittle bone disease.

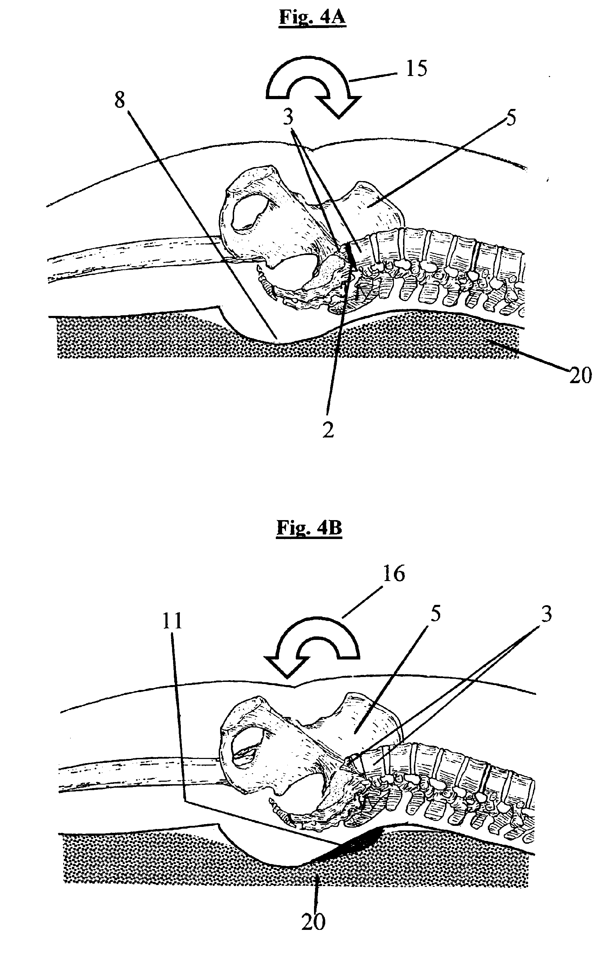

Referring to Fig. 4A the natural pelvis 5 rotation 15 when the body is in the sleeping (back) position is caused by the unbalanced pressure exerted by the predominant gluteus maximus (buttocks) 8 which have a pressure point on the opposite side of the pelvis 5 centre line from the spine. Again the pelvis 5 naturally rotates and the misalignment is absorbed by the transitional vertebrae 3 resulting in the pinching of cartilage 2 and nerves at the front of the body and the stretching to the limit of the ligaments at the back of the body.

Referring to Fig. 4B, the pelvis 5 is shown in its properly aligned position 16 as a result of wearing the invention 11 when the body is in the sleeping (back) position. Once again the belt 11 is designed to take pressure from the mattress 20 in order to provide the support.

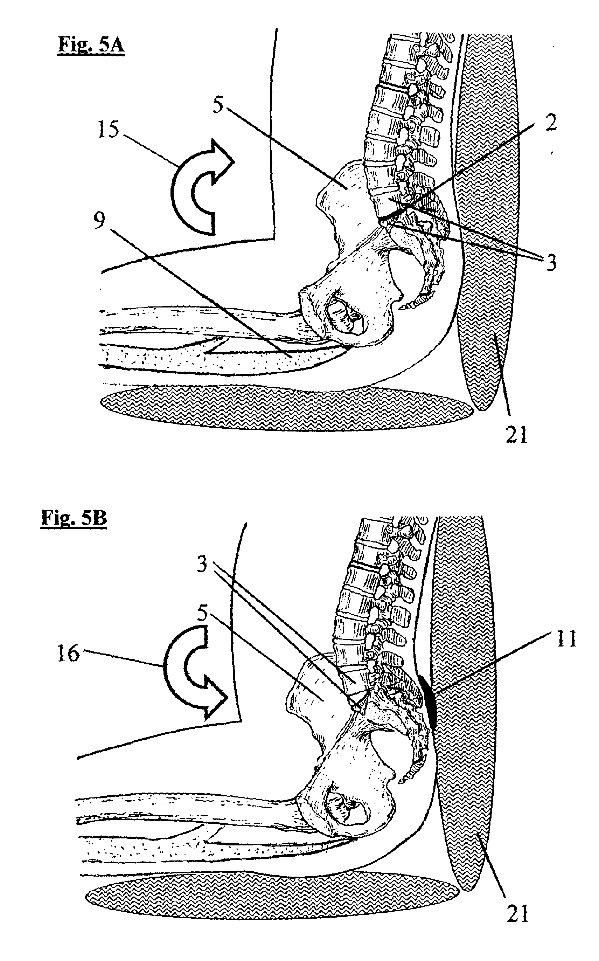

Referring to Fig. 5A, the pelvis 5 rotation 15 when the body is in a sitting position, partly caused by the hamstring muscles 9 in the back of the thighs is shown. These are the strongest set of muscles in the body responsible for the power when walking and running. In the sitting position, the hamstrings 9 in both legs cause natural rotation 15 of the pelvis 5 backwards resulting in misalignment of the transitional vertebrae 3. As with the sleeping (back) mode, the result is pinching of the cartilage 2 and nerves at the front of the vertebrae and the stretching to the limit of the ligaments at the back of the vertebrae.

Referring to Fig. 5B the pelvis 5 is shown in its properly aligned position 16 due to the presence of the invention 11 when the body is in a sitting position by taking the pressure from the seat back 21. It is important that the wearer of the day-time wedges is conscious of the importance of chair back 21 designs for providing the correct support. Chairs which only have back support at a high level (at or above the waist) should be avoided as this totally nullifies the effect of the invention. With or without the wedges these chairs can cause damage to the back in the transitional vertebrae 3 region and should be avoided. Furthermore, sitting on stools or benches with no backs has been the cause of a considerable amount of spinal damage and should be avoided especially by people vulnerable to this type of complaint.

A common feature of the pelvis support belts for both the sleeping and sitting positions is that they rely on a co-operating support for their correct operation. Referring to Figs. 3A/B and 4A/B, in the sleeping position the belt is designed to take pressure from the mattress 20 in order to provide the support. In the sitting position in Figs. 5A/B the belt is designed to take pressure from the back of the seat 21 in order to provide the support. In both cases, the presence of the mattress 20 or seat back 21 is consistent with the relaxing of the muscles in the body and hence the need for the invention.

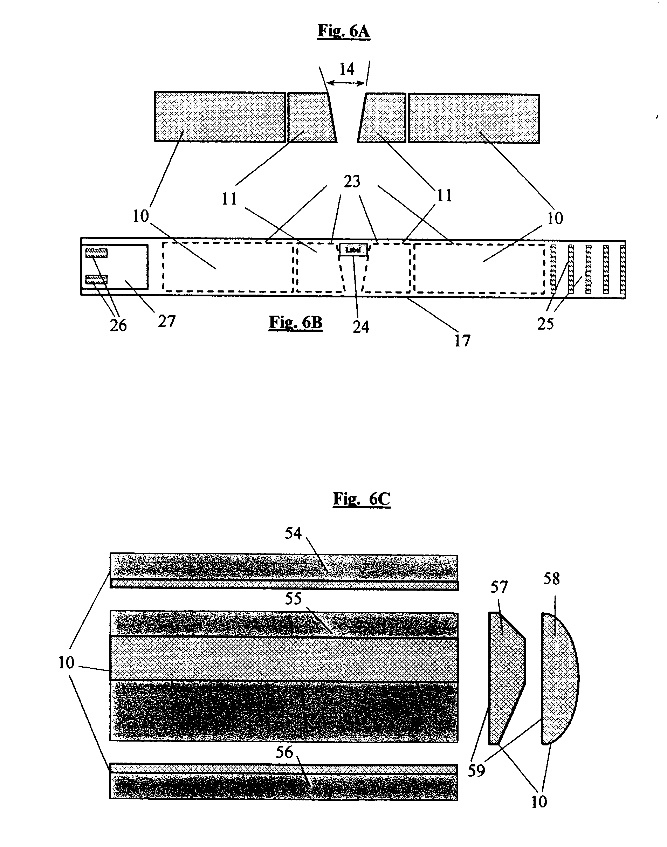

Referring to Figs. 6A and 6B the design of the pelvis support belt 17 is such as to hold the wedges 10 and 11 in position with relation to each other. Each side wedge 10 and corresponding rear wedge 11 are assembled next to each other while the pair of rear wedges 11 have a specific gap 14 between them. The gap 14 is tapered with the larger distance between them at the top. A label 24 is fixed in this wider space at the top of the belt 17 as a guide so that the wearer does not mistakenly wear the belt 17 up-side down. The label 24 contains the name and other information about the garment.

Referring to Figs. 6C to 6E the shapes of the support wedges 10 and 11 vary in criticality. Fig. 6C showing different views of a single side wedge 10 illustrates how it can be a simple shape being a consistent wedge profile along its whole length. The top view 54, side view 55 and bottom view 56 are consistent with the end view 57. As illustrated by Figs. 3A and 3B the side wedges are used to counteract the high point pressure 7 of the Femur (thigh bone) 6. The wedge angle 57 and whether or not it goes to a point is not critical. However, the average thickness requirement will vary from one person to another depending on body and bone sizes. In Fig. 6C the face 59 of the wedges 57 and 58 against the body will be flat while the outside face may be angled 57 or curved 58.

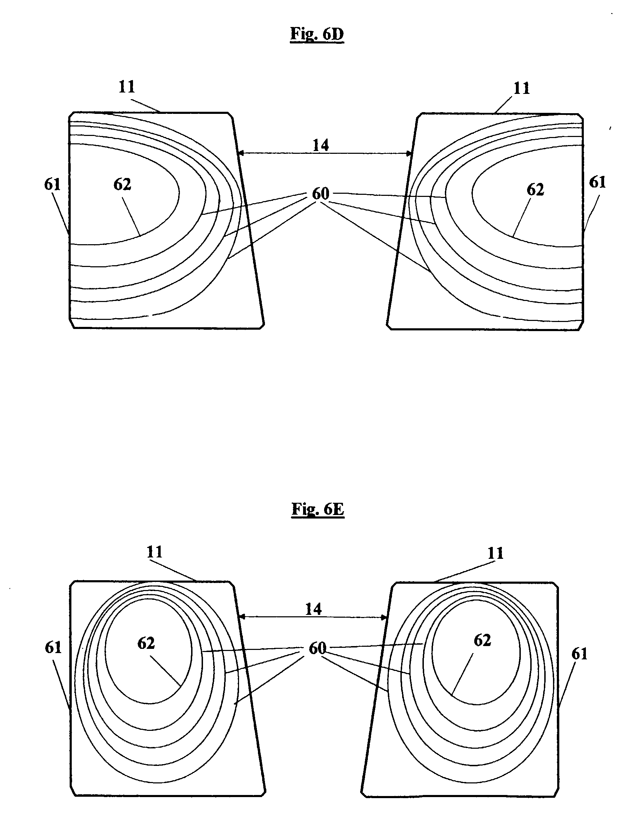

Referring to Figs. 6D and 6E the outside faces of two pairs of rear wedges 11 for which the dimensions are more critical. The inside face of each wedge 11 which is against the body is flat while the outside is shaped as depicted by the contour lines 60 showing the variation in thickness. Typical thicknesses are 0.5 centimetres at the edges and 2.0 centimetres at the centre contour. These dimensions and the gap 14 dimensions between the wedges 11 are quite critical. The two main functions of the rear wedges 11 are to rotate the pelvis 5 forwards and to ensure that there is no pressure on the spine. The size and shape of the gap 14 is such that there is no pressure on the sacrum 4 or lumbar vertebrae 1. It is also important that, in addition to wearing the support wedges 11, there is no lump in the clothing such as a button, trouser belt loop or bracer clip which presses on the sacrum 4 or the lumbar vertebrae 1.

It is also essential that the pressure from the rear support wedges 11 on the pelvis 5 is evenly distributed. Referring to Fig. 1 the pelvis 5 and the sacrum 4 are fused together. The pelvis 5 is made of two mirror image halves joined together at the base and with the upper halves joined to the sacrum 4. If the support wedges 11 are too thick at the outside edges they will put too much pressure on the outsides of the pelvis 5 and the two halves of the pelvis 5 will try to fold inwards and start to bend the joints where they are joined at the base and where they are attached to the Sacrum 4. Conversely, if the inside edges of the rear wedges 11 are too thick it will try to bend the pelvis 5 in the opposite direction. Distortion of these joints for long periods while sitting or sleeping may cause discomfort.

Fig. 6D shows the night time wedges 11 with part of the outside edges 61 equal in thickness to their thickest part. This provides continuity of thickness through to the side wedges 10 in Fig. 6C. Fig. 6E shows the day-time wedges 10 which have the outside edges 61 tapered back down to the smallest thickness. This ensures that ugly projections of the corners do not show through day-time clothes and so the invention can be worn inconspicuously.

Referring to Figs. 6D and 6E an alternative design uses a larger area for the rear wedges especially for invalids who spend most of their time lying or sitting in one position. This provides more comfort at the expense of elegance. In some circumstances the wedges can be made-to-measure to provide an exact fit for an individual person. The basic shape shown in Figs. 6D and 6E is also not critical as the edges may be curved and the comers rounded. The whole wedge 11 is shown to be wider at the bottom than the top so that a pocket of the same shape will naturally hold the wedge in place.

The wedges may be made of extruded, moulded or sculpted expanded polymer such as polyethylene or similar material. The side wedges 10 are flexible enough to easily bend around the shape of the hips yet firm enough to not distort and lose their wedge shape and hence their support qualities. The choice of wedge material is a compromise between comfort and compactness. In order to be aesthetically acceptable, the belt must not protrude excessively from the user's body. The pelvis support wedges can additionally be either perforated or ridged for additional user comfort, allowing the skin to breath. The support wedges can be impregnated with a therapeutic compound whereby the vapours produced protect the skin from dermatological or other problems such as body odour which might result from wearing the invention.

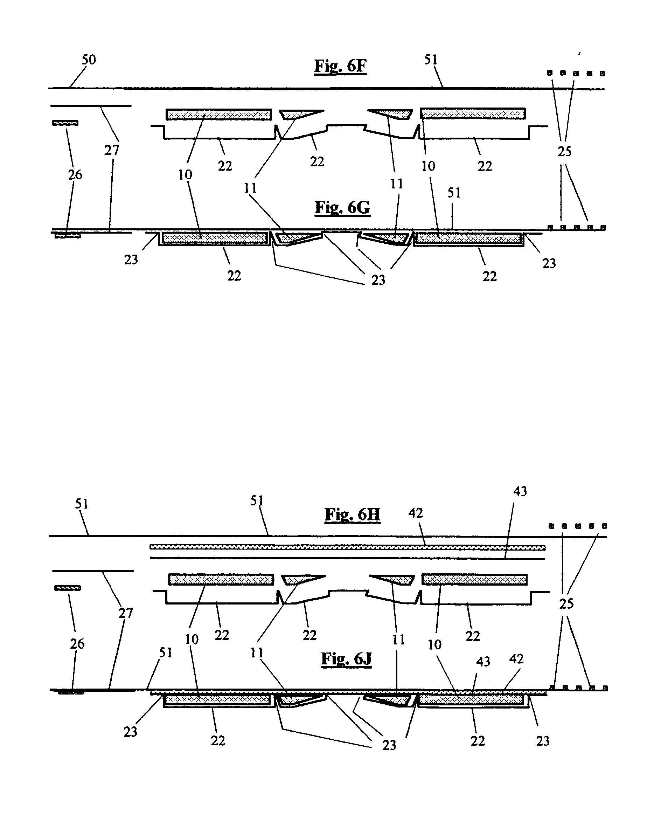

Referring to Figs. 6F to 6J the components of two examples of the invention and how they go together are shown viewed from above. Fig. 6F is a top view of the simplest form of the invention with the different components shown in exploded view. The strip 51 next to the skin is non-slip, non-elastic cotton winceyette or similar fabric which prevent the wedges 10 and 11 moving around with respect to the pelvis 5. The strip 51 material is normally made from natural fabrics to provide maximum comfort and to avoid irritation the skin and not produce allergic reactions in the user. However, the materials making up the belt can be of other materials including paper type materials which would be low cost and so be classed as disposable especially for use in hospitals where they may easily be soiled and not convenient to keep clean and sanitary. The wedges 10 and 11 are held to this first layer of fabric 51 by a piece of the same or similar material 22 sewn 23 into pockets, as a single piece as shown or separately as several pieces. Fig. 6H shows the same view as Fig. 6F but has two additional layers of material, firstly a thicker wadding type material 42 frequently used in the clothing industry to add comfort and to allow the skin to breathe. Secondly a layer of winceyette 43 is sewn in a quilted or similar fashion to the first layer through the wadding to keep the latter in place. Figs. 6F and 6H also show elastic strips 27 and the fastening strips 25 and 26. The elastic 27 provides the "give" in the belt to allow body movement and expansion of the hips without discomfort. Fasteners 25 and 26 where the two ends of the belt come together at the front of the body may be Velcro™ strips, buttons, hooks and eyes or tie chord. To avoid discomfort there is a continuation of the cotton fabric 50 between the body and the fastener where it is attached to the elastic 27 . Alternatively instead of providing a means of fastening the belt at the front a continuous band of the fabric with elastic inserts around the body may be used.

Figs. 6G and 6J show all the components as in Figs. 6F and 6H respectively but in a non-exploded view as they would be sewn together. The points where the pockets 22 are sewn 23 to the belts are indicated.

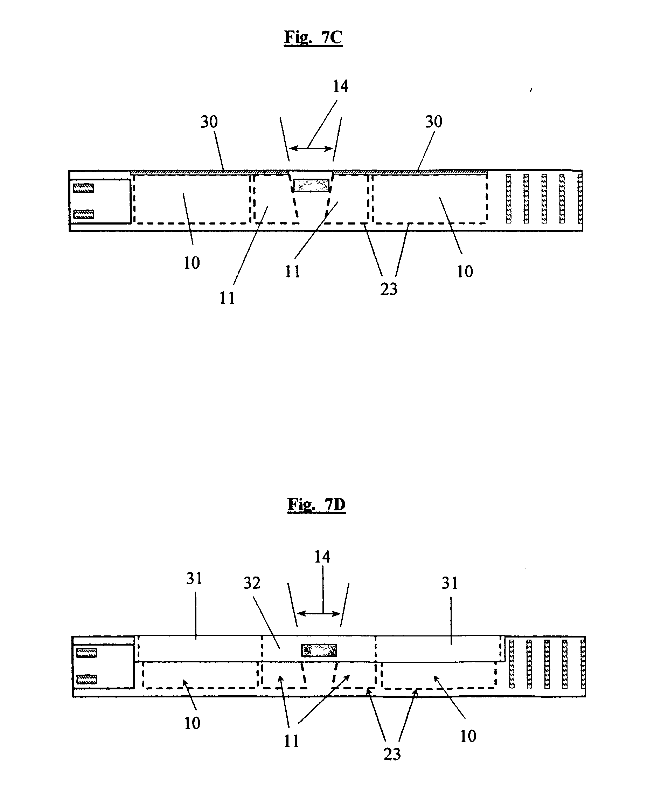

Alternative designs of the invention are shown in Figs. 7A to 7D. Fig. 7A is the day-time equivalent of the belt 17 shown in Fig. 6B. The day-time belt has no side wedges. The shape and profiles of the rear wedges 11 are similar to the night-time belt except that they avoid protruding corners which show through the clothes.

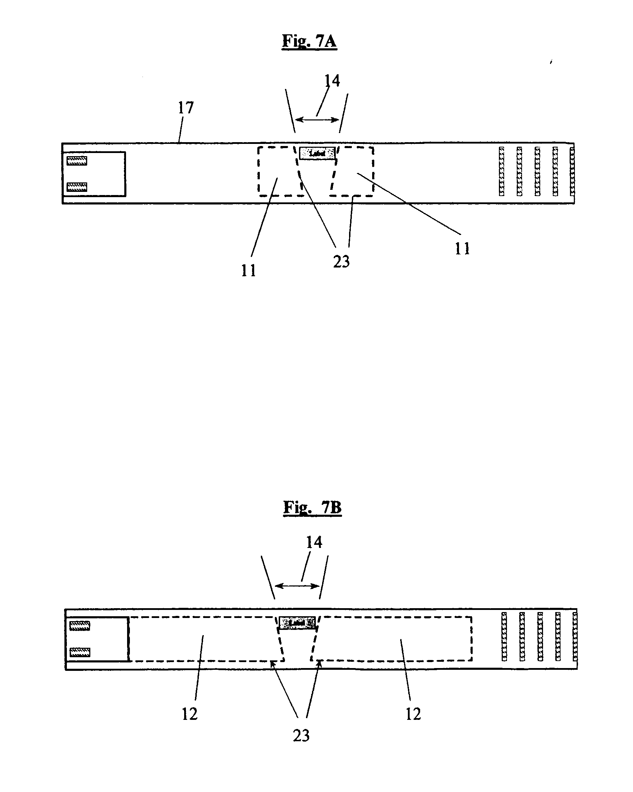

Fig. 7B is a night-time belt 17 identical to the one in Fig. 6B except that adjacent side and rear wedges 12 are formed as a single integral wedge. The extra complication of forming the integrated wedge may be compensated by the reduction of the total number of pockets to only two.

Fig. 7C is a night-time belt 17 identical to the one in Fig. 6B except that the tops 30 of the pockets 23 are not sewn together but held together by some fastening method such as Velcro™ or zipper. This allows removal of the wedges 10 and 11 for the purpose of washing the rest of the belt to preserve the wedges from damage in the washing machine. It also allows the belt to be supplied with several thickness' of wedge so that the user can experiment to find the most suitable size. Furthermore, the side wedges 10 can be removed to convert the night-time belt into a day-time belt. An alternative design to having the fourth side of the pockets held closed by Velcro™ is to leave them open but make the pockets of elastic material which will stretch to allow insertion of the wedges and then grip them to hold them in place. Taking this a next step, the whole belt may be made from elastic material.

Fig. 7D is a night-time belt identical to the one in Fig. 7C except that instead of Velcro™ holding together the fourth side 30 of the pockets 23, fabric flaps 31 over the tops of the pockets 23 keep the wedges 10 and 11 in place. An alternative design is to have the pockets detachable whereby the pockets containing the wedges are held in place on the belt by Velcro™ or a similar means and may be detached with the wedges inside for the purpose of washing the rest of the belt.

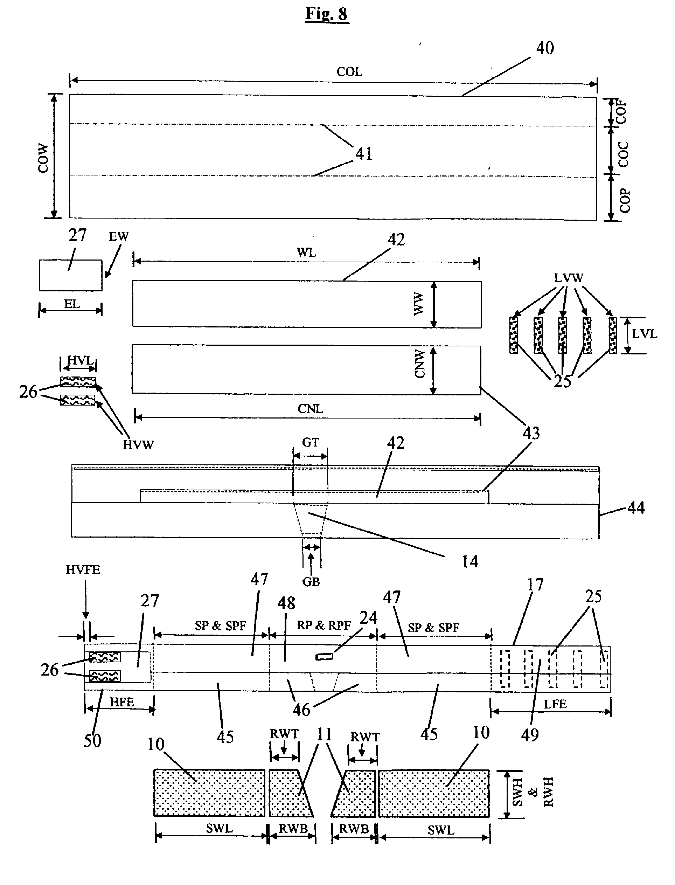

Fig. 8 shows the stages of manufacture of a typical example of the invention, with reference to Table 1:

**Table 1**

| Dimensions of Medium-Size Belt as referenced in Fig. 8 | | | | |
|---|---|---|---|---|
| **Code** | **Dimension** | **CM** | **Ref. No.** | **Component** |
| COL | Length | 122 | 40 | Cotton outer fabric |
| COW | Width | 30 | 40 | Cotton outer fabric |
| COP | Pocket | 11 | 40 | Cotton outer fabric |
| COC | Center | 12 | 40 | Cotton outer fabric |
| COF | Flap | 7 | 40 | Cotton outer fabric |
| WL | Length | 82 | 42 | Wadding |
| WW | Width | 12.5 | 42 | Wadding |
| CNL | Length | 82 | 43 | Cotton inner fabric |
| CNW | Width | 12.5 | 43 | Cotton inner fabric |
| EL | Length | 15 | 27 | Elastic |
| EW | Width | 8 | 27 | Elastic |
| HVL | Length | 8 | 26 | Hook Velcro ™ |
| HVW | Width | 2.5 | 26 | Hook Velcro™ |
| HVFE | From end | 1 | 26 | Hook Velcro™ |
| LVL | Length | 9 | 25 | Loop Velcro™ |
| LVW | Width | 1.25 | 25 | Loop Velcro™ |
| GT | Top | 8 | 14 | Tapered gap |

| Code | Dimension | CM | Ref. No. | Component |
|---|---|---|---|---|
| GB | Bottom | 4 | 14 | Tapered gap |
| SP | Length | 26 | 45 | Side pockets |
| RP | Length | 28 | 46 | Rear pockets |
| SPF | Length | 26 | 47 | Side pocket flaps |
| RPF | Length | 28 | 48 | Rear pocket flaps |
| LFE | Length | 25 | 49 | Loop fastener end |
| HFE | Length | 15 | 50 | Hook fastener end |
| SWL | Length | 22 | 10 | Side wedges |
| SWH | Height | 11 | 10 | Side wedges |
| SWTH | Thickness | 2 | 10 | Side edges (note 1) |
| RWT | Top | 8 | 11 | Rear wedges |
| RWB | Bottom | 10 | 11 | Rear wedges |
| RWH | Height | 11 | 11 | Rear wedges |
| RWTH | Thickness | 2 | 11 | Rear wedges (note 1) |
| Note 1 - dimension not illustrated in Fig. 8. | | | | |

Table 1 shows the dimensions of a medium sized belt referenced by the codes shown in Fig. 8. The large piece 40 of winceyette or similar is 122cm long and 30cm wide and will fit someone with a hip measurement of between 95 and 115cm. The fold lines 41 are to form the pockets 45 and 46 from the bottom and flaps 47 and 48 from the top. A piece of wadding 42 provides a soft layer in the area where there are wedges. A piece of winceyette 43 holds the wadding in place. Elastic 27 and Velcro™ strips 25 and 26 provide the method of fastening and the necessary "give" to allow body movement. The label 24 which may be directly embroidered into the fabric 40 gives information about the belt and shows which way up the belt must be worn.

Fig. 8 further shows where the wadding 42 (behind) and retaining winceyette 43 are first sewn onto the main piece 44 of winceyette 40 along the edge with the bottom section of the main piece folded up to form the pockets and the centre tapered gap 14 stitched. Fig. 8 also shows the completed belt 17 with the pockets 45 and 46 and flaps 47 and 48 stitched. The rear wedges 11 are shown tapered with the widest part at the bottom which helps to hold them firmly in their pockets 46. The flap 48 over the rear wedges is not stitched on the two inside edges and so makes it easier to remove the wedges when required. The label 24 is placed on the outside of the belt 17 at the top in between the rear wedge pocket 46. This helps to ensure that the belt is worn the correct side upwards. The label 24 is not placed on the inside of the belt 17 as it could irritate the skin. The sides of the pockets 47 for the side wedges 10 are shown sewn vertically although they could be tapered to help retain the wedges. The "loop" Velcro™ 25 on the inside of the belt is on the right hand end. The "hook" Velcro™ 26 is on the outside of the belt 17 away from the skin. The latter is also attached to the elastic strip 27. The elastic is sewn both ends with winceyette between it and the skin to avoid irritation and to keep the "hook" part of the Velcro™ away from pubic hair as this can cause distress.

An alternative to the pelvis support belt is the pelvis support garment, specifically shorts, pants, pyjamas or other garment worn around the hips. Clearly the style of garment can be chosen as applicable dependent on age, gender and so forth. The belt as an integral part of a garment has several advantages over the belt by itself: the combined arrangement is more efficient for stopping the belt from riding up the body, it is more elegant, it is more comfortable and it is more convenient. The support wedges are provided as an integral part of the garment such that they will be located around the top part of the pelvis. The method of attaching the wedges to the garment may be similar to any of the methods previously described with respect to the pelvis support belt. The garment may be fastened by any of the means described for the belt or may have a continuous waistband with elastication.

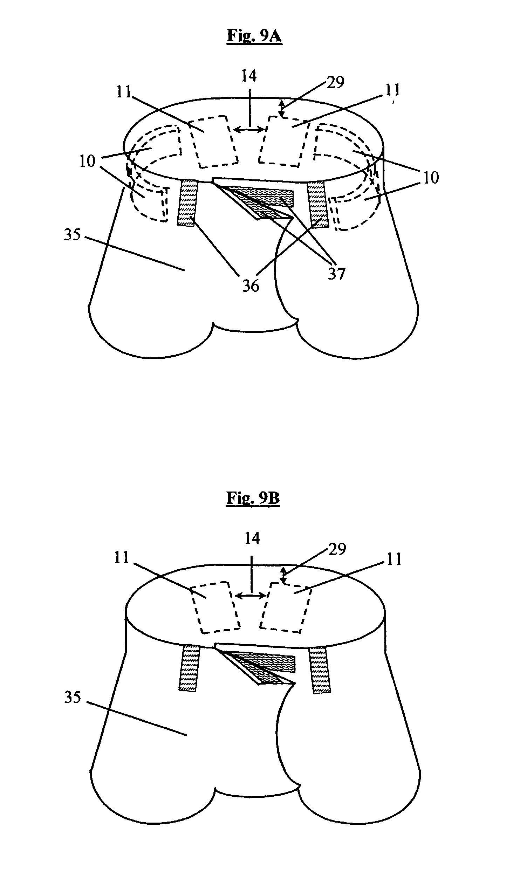

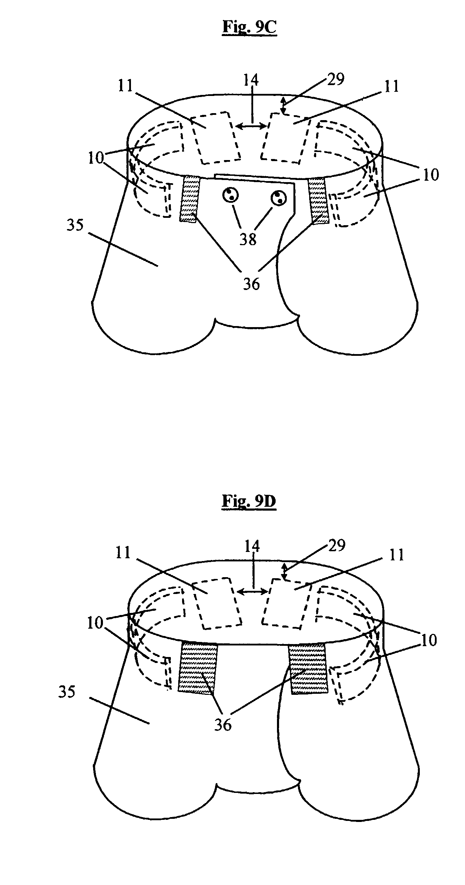

Four variations of the invention in the style of "boxer" shorts are shown in Figs. 9A to 9D. Fig. 9A shows a pair of night-time pelvis support shorts 35 essentially equivalent to the belt 17 shown in Fig. 6B. The wedges 10 and 11 are shown sewn into four pockets although any of the methods of retaining the wedges previously given for the belt version of the invention may also be applied. The waistband of the shorts contains two elastic inserts 36 to allow natural movement of the body and is fastened together at the front by Velcro™ 37. Fig. 9B shows the day-time equivalent to Fig. 9A. Fig. 9C is the same as Figs. 9A except the front is fastened by buttons 38 instead of Velcro™. Fig. 9D is the same as Fig. 9A except that the top of the shorts is continuous with just elastic inserts 36 in a continuous waistband.

The dimensions, spacing and so forth of the various elements in Figs. 9A to 9D are the same as that indicated in Fig. 8 and Table 1. In Figs. 9A to 9D a spacing 29 of 6cm is allowed between the top of the shorts and the wedges.

The invention is designed to maintain the position of the support wedges with respect to the pelvis in spite of various movements of the body such as turning over in bed or the multitude of movements during the day. To prevent it from sliding downwards or around the body, the belt is made of a firm material with a minimum amount of stretch. To prevent the belt from riding upwards the material in the garment under the crutch and the grip of the leg sections around the thighs is designed for this purpose. Separate shorts could also provide the latter functions over the top of the belt version of the design although this will not be as efficient. One further benefit of the day-time invention is that it provides extra support for trousers worn over the top and makes trouser belts or braces essentially redundant. At the same time, the trousers also help stop the invention riding upwards. The day-time version of the invention for women may be sculpted in such a way as to enhance the overall shape of the profile of their buttocks

Two further advantages of making the pelvis support belt an integral part of a garment are comfort and aesthetics which may be important if the assembly is to be a full time, permanent feature for the wearer. The condition of low level inflammation in the lower back is a common complaint. Most people with the complaint will have consulted doctors but ended up frustrated with the impression that there is no real cure. They may have been recommended various back support devices such as the body corset. In the case of the corset, it is likely that it will have failed because, not only is it an uncomfortable clumsy device, it also makes the wearer look and feel like an invalid which inevitably leads to further stress. Furthermore, a corset relies on being strapped very tightly around the abdomen and can cause stomach problems such as an hiatus hernia. It is quite common for the corset device to only be available subject to a doctor's approval. Finally, the corset principle suppresses the natural flexibility of the spine all the time it is being worn. By contrast the invention is designed for maximum comfort and functions without interfering with the operations of any other part of the body as it only applies pressure when and where it is needed, namely in the relaxed sleeping and sitting positions.

The aesthetic appeal of the invention is important, which is why it may be incorporated within stylish garments. As a result of the careful design, the presence of the pelvis support garment will go totally unnoticed by a "passer-by" in the case of the day-time-sitting version and will not be conspicuous in the case of the night-time-sleeping model. It is essential that the invention is accepted as a routine everyday garment because in most cases it will be needed every day and night for the rest of the wearer's life. The first phase of wearing the garments will be to promote repair of the damaged back, after which the back will always need its protection.

As discussed above, the support wedges are made of a flexible material such as extruded, moulded or sculpted expanded polyethylene or other polymer or other similar material. A naturally occurring material would be preferable from an ecological point of view but the specifications are challenging. The material is preferably resistant to damage when being washed and dried in domestic clothes washing and drying machines, to retain its shape and integrity for a certain minimum number of washing and drying cycles. Thus the version of the invention which allows removal of the wedges for laundering allows for a wider choice of materials. The compromise between comfort and compactness is important. It is essential that the wearer is not constantly conscious of the invention if it is to be worn for the rest of his or her life. While greater compressibility provides more comfort it also protrudes more and is more likely to be conspicuous.

It is anticipated that the invention can be used in conjunction with some other body support device. One such device might be a standard lumbar region support which would be appropriate to invalids who spend all their time in the lying and or sitting position.

The overall size of the invention varies depending on the size of the wearer. However, some variation in size is provided by some types of front fastening. With Velcro™ strips long enough to adjust for a wide range of hip measurements a limited number of sizes is able to cover the complete range of requirements.

## Claims

1. A pelvis support for a wearer comprising one or more support elements and a retainer for retaining the support elements on the wearer's body, in which the support element is arranged to co-operate with an external surface to support the wearer's body therapeutically.

2. A pelvis support as claimed in claim 1 in which the retainer is arranged to retain the support element in a pelvis supporting position.

3. A pelvis support as claimed in claim 1 or 2 in which the or each support element is approximately wedge-shaped, tapering downwards in use.

4. A pelvis support as claimed in any preceding claim in which the retainer includes side portions and a back portion arranged in use, to pass around the sides and back respectively of the user and preferably in which the support element is provided at the side portions and back portion of the retainer and/or the or each support element is provided at the back of the retainer and preferably in which the support element is interrupted at the back portion of the belt to provide clearance, in use, for the wearer's spine.

5. A pelvis support as claimed in any preceding claim in which the or each support element is removably mounted in the retainer.

6. A pelvis support as claimed in any preceding claim in which the retainer comprises a belt or in which the retainer comprises a garment for the wearer, for example an item of nightwear.

7. A pelvis support as claimed in any preceding claim in which the retainer includes a layer of firm material extending around at least the inside portion of the support elements and preferably in which a layer of soft material is provided at the innermost part of the retainer between the support elements and the user for increased comfort.

8. A pelvis support as claimed in any preceding claim in which the support elements are sewn into the retainer or in which the or each support element is retained in a pocket provided in the retainer and preferably in which each pocket is configured to allow the insertion of one or more support elements to achieve the desired thickness.

9. A pelvis support as claimed in any preceding claim in which the support element comprises a padding element.

10. A pelvis support as claimed in any preceding claim partially or wholly constructed of disposable materials and preferably impregnated with fragrant material and preferably in which at least one support element comprises a substantially flat wearer support face and a contoured opposing face including a raised outer, upper region tapering to a thinner, lower inner portion relative, in use, to the wearer's spine and preferably comprising a wedge-shaped insert.
